# EUROPÄISCHE PATENTSCHRIFT

(11) **EP 1 483 413 B1**
(45) Veröffentlichungstag und Bekanntmachung des Hinweises auf die Patenterteilung: **05.10.2005**
(21) Anmeldenummer: 03714829.3
(22) Anmeldetag: 14.03.2003
(51) Int. Cl.: C12Q 1/68

(54) **VERWENDUNG VON ABFANGSONDEN BEIM NACHWEIS VON NUKLEINSÄUREN**
USE OF CAPTURING PROBES FOR IDENTIFYING NUCLEIC ACIDS
UTILISATION DE SONDES DE CAPTURE POUR IDENTIFIER DES ACIDES NUCLEIQUES

(30) Priorität: 14.03.2002 DE 10211321
(43) Veröffentlichungstag der Anmeldung: 08.12.2004
(73) Patentinhaber: Gnothis Holding SA, 1015 Ecublens (CH)
(72) Erfinder: KORN, Kerstin, S-11825 Stockholm (SE)
(74) Vertreter: Weiss, Wolfgang
(86) Internationale Anmeldenummer: PCT/EP2003/002713
(87) Internationale Veröffentlichungsnummer: WO 2003/076656

(56) Entgegenhaltungen:
- US-A- 5 780 224
- US-A- 6 103 476
- US-B1- 6 280 952
- CANTOR C. R.: "LIGHTING UP HYBRIDIZATION" NATURE BIOTECHNOLOGY, Bd. 14, 1. März 1996 (1996-03-01), Seite 247 XP002094958 ISSN: 1087-0156

## Beschreibung

Die Erfindung betrifft ein Verfahren zur Bestimmung eines Nukleinsäure-Analyten in einer Probe unter Verwendung einer mit den Analyten bindefähigen Hybridisierungssonde und einer mit der Hybridisierungssonde bindefähigen Abfangsonde. Weiterhin wird ein Hybridisierungs- und Abfangsonden enthaltender Reagenzienkit bereitgestellt. Verfahren und Reagenzienkit können insbesondere für die Bestimmung von Analyten eingesetzt werden, die in der Probe nur in sehr geringen Konzentrationen vorkommen.

Die Verwendung von Hybridisierungssonden zum Nachweis von Nukleinsäure-Zielmolekülen als Analyten in einer Probe ist seit langem bekannt. Als Hybridisierungssonde wird üblicherweise eine mit einer Teilsequenz des Analyten komplementäre Sonde aus Nukleotid- oder Nukleotidanalogon-Bausteinen verwendet, die spezifisch mit dem in der Probe vorhandenen Analyten bindet und eine nachweisbare Markierungsgruppe trägt.

Ein besonders sensitives Verfahren zum Nachweis von Analyten, auch Nukleinsäure-Analyten, ist die sogenannte Fluoreszenz-Korrelationsspektroskopie (FCS), die beispielsweise in EP-B-0 679 251 beschrieben ist. Diese Methode erlaubt den Nachweis einer nur sehr geringen Anzahl von Molekülen in der Probe bis hinab zum Nachweis von Einzelmolekülen.

Ein - generell bei Hybridisierungsverfahren, im besonderen Maße jedoch bei hochsensitiven Verfahren, wie etwa FCS, bestehendes - Problem ist ein Hintergrundsignal, das durch nicht an den Analyten gebundene markierte Hybridisierungssonden erzeugt wird. Die Intensität dieses Hintergrundsignals wird weiterhin durch das Vorhandensein eines Überschusses von markierten Hybridisierungssonden gegenüber den in der Probe vorhandenen Analytmolekülen erhöht, der in vielen Fällen, beispielsweise aus kinetischen Gründen oder bei der quantitativen Bestimmung von Analyten, notwendig ist.

Zur Verringerung des von nicht gebundenen Hybridisierungssonden stammenden Signals können sogenannte Molecular Beacons eingesetzt werden, die üblicherweise eine Hairpin-Struktur, bestehend aus einem Stem mit zwei zueinander komplementären Sequenzabschnitten und einem Loop, eine Fluoreszenzmarkierungsgruppe und eine Quenchgruppe enthalten. Die nicht mit dem Zielmolekül bindefähige Stem-Sequenz dient dazu, die Fluoreszenzmarkierungsgruppe und die Quenchgruppe in Nachbarschaft zu halten, wenn die Sonde nicht an den Analyten gebunden ist. Auf diese Weise werden Photonen, die durch die Fluoreszenzmarkierungsgruppe absorbiert werden, nicht als Fluoreszenzphotonen emittiert, sondern die Energie wird auf die Quenchgruppe übertragen. Die Loop-Sequenz ist mit den nachzuweisenden Zielmolekül komplementär. Bei Bindung der Sonde an das Zielmolekül öffnet sich der Loop und hybridisiert mit dem Zielmolekül, so dass die Fluoreszenzmarkierungsgruppe aus der Nachbarschaft der Quenchgruppe entfernt wird. Dadurch kann Fluoreszenz auftreten und gemessen werden.

Im US-Patent 5,780,224 wird die Verringerung des von nicht gebundenen Hybridisierungssonden stammenden Signals dadurch erreicht, daß ein Überschuß von mindestens einer Hybridisierungssonde eingesetzt wird, was zu einer Komplexbildung führt. Danach werden die ungebundenen Hybridisierungssonden über eine Abfangsonde an ein Partikel oder Ähnliches gebunden und mit dem Partikel zusammen entfernt.

Eine der vorliegenden Erfindung zugrunde liegende Aufgabe bestand somit darin, die oben genannten Nachteile bei einem Verfahren zur Bestimmung von Nukleinsäure-Analyten zumindest teilweise zu vermeiden.

Diese Aufgabe wird dadurch gelöst, dass ein Hintergrundsignal, das von nicht an den Analyten gebundenen Markierungsgruppen in der Probe stammt, durch Verwendung einer Abfangsonde, die an der Hybridisierungssonde binden kann, aber nicht damit kovalent assoziiert ist, selektiv abgeschwächt bzw. verringert wird. Dies führt zu einer Verbesserung des Verhältnisses zwischen dem Messsignal, das von einer an den Analyten gebundenen Markierungsgruppe stammt, und dem Hintergrundsignal, die vorzugsweise mindestens einen Faktor von 2 und besonders bevorzugt mindestens einen Faktor von 5 beträgt. Hierzu wird eine Kombination, umfassend eine Hybridisierungssonde und eine mit der Hybridisierungssonde bindefähige Abfangsonde eingesetzt, wobei die Abfangsonde eine Abfanggruppe trägt, und durch die Bindung an eine freie, d.h. nicht analytgebundene, Hybridisierungssonde eine Verringerung des Signals von deren Markierungsgruppe bewirkt. Um die Bindung der Hybridisierungssonde an den Analyten nicht zu beeinträchtigen, müssen Hybride zwischen Abfangsonden und Hybridisierungssonden einen geringeren Schmelzpunkt als Hybride zwischen Analyten und Hybridisierungssonden aufweisen.

Ein Gegenstand der Erfindung ist somit ein Verfahren zur Bestimmung eines Nukleinsäure-Analyten in einer Probe, umfassend die Schritte:
(a) Bereitstellen einer Probe, die den Analyten enthalten kann,
(b) Bereitstellen von mindestens einer mit dem Analyten bindefähigen Hybridisierungssonde, die eine Markierungsgruppe trägt,
(c) Bereitstellen von mindestens einer mit der Hybridisierungssonde bindefähigen Abfangsonde, wobei die Abfangsonde mindestens eine Abfanggruppe trägt, die bei Bindung an die Hybridisierungssonde eine Verringerung des von der Markierungsgruppe stammenden Signals bewirkt und wobei ein Hybrid zwischen Abfangsonde und Hybridisierungssonde einen geringeren Schmelzpunkt als ein Hybrid zwischen Analyt und Hybridisierungssonde aufweist,
(d) Inkontaktbringen von Probe und Hybridisierungssonde unter ersten Hybridisierungsbedingungen, bei denen ein stabiles Hybrid zwischen Hybridisierungssonde und Analyt entstehen kann, aber bei denen kein stabiles Hybrid zwischen Hybridisierungssonde und Abfangsonde entstehen kann,
(e) Inkontaktbringen von Probe, Hybridisierungssonde und Abfangsonde unter zweiten Hybridisierungsbedingungen, bei denen ein stabiles Hybrid zwischen nicht an den Analyten gebundener Hybridisierungssonde und Abfangsonde entstehen kann und wobei durch Bindung der Hybridisierungssonde an die Abfangsonde eine Verringerung des von deren Markierungsgruppe stammenden Signals bewirkt wird, und
(f) Nachweisen der Bindung der Hybridisierungssonde an den Analyten.

Die durch das Verfahren bestimmten Nukleinsäure-Analyten, z.B. DNA oder/und RNA, können aus biologischen Proben, insbesondere aus Säugern wie dem Menschen, aber auch anderen Organismen, z.B. Mikroorganismen, stammen. Darüber hinaus können auch Analyten nachgewiesen werden, die *in vitro* aus biologischen Proben erzeugt worden sind, z.B. cDNA-Moleküle, die durch reverse Transkription aus mRNA hergestellt worden sind. Die für das Verfahren verwendete Probe stammt vorzugsweise aus einem Organismus, z.B. aus Gewebe oder Körperflüssigkeit eines Organismus, insbesondere aus einem Menschen.

Das Verfahren kann beispielsweise zu einer Analyse der Genexpression, z.B. zur Ermittlung eines Genexpressionsprofils oder zur Analyse von Mutationen, z.B. Einzelnukleotidpolymorphismen (SNP), eingesetztwerden. Das Verfahren eignet sich jedoch auch zur Bestimmung enzymatischer Reaktionen an Nukleinsäuren, beispielsweise zur Bestimmung von Nukleinsäureamplifikationsreaktionen, insbesondere in einem oder mehreren Thermocycling-Prozessen.

Die für das erfindungsgemäße Verfahren eingesetzten Hybridisierungssonden sind vorzugsweise Oligonukleotide, insbesondere DNA-Moleküle. Es können jedoch auch entsprechende Nukleinsäureanaloga, wie etwa Peptidnukleinsäuren (PNA), Locked Nukleinsäuren (LNA) oder andere Nukleotidanaloga, eingesetzt werden. Die Länge der Hybridisierungssonden kann grundsätzlich wie bei bekannten Sonden gewählt werden und liegt üblicherweise im Bereich von 15 bis 200 Nukleotiden.

Die Hybridisierungssonden tragen eine Markierungsgruppe, die ein durch geeignete Methoden nachweisbares Signal erzeugen kann. Vorzugsweise erzeugt die Markierungsgruppe ein durch optische Methoden nachweisbares Signal. Besonders bevorzugt ist die Markierungsgruppe eine Fluoreszenzmarkierung, wie etwa Fluorescein, Rhodamin, Phycoerythrin, CY3, CY5 oder Derivate davon.

Ein wesentliches Merkmal des erfindungsgemäßen Verfahrens ist die Verwendung von Abfangsonden, die eine Abfanggruppe tragen. Die Abfangsonden können unter geeigneten Hybridisierungsbedingungen an die Hybridisierungssonden binden, sie sind jedoch nicht damit kovalent assoziiert. Die Abfangsonden sind - ebenso wie die Hybridisierungssonden - vorzugsweise Oligonukleotide, insbesondere DNA-Moleküle. Es können jedoch auch entsprechende Nukleinsäureanaloga, wie oben genannt, eingesetzt werden. Die Länge der Abfangsonden kann entsprechend der Länge der Hybridisierungssonden gewählt werden.

In einer ersten bevorzugten Ausführungsform kann als Abfanggruppe eine das Signal der Markierungsgruppe, beispielsweise einer Fluoreszenzmarkierungsgruppe, zumindest teilweise löschende Quenchgruppe verwendet werden. Wenn eine Quenchgruppe in räumlicher Nachbarschaft zu einer Fluoreszenzmarkierungsgruppe vorhanden ist, werden Photonen, die durch die Fluoreszenzmarkierungsgruppe absorbiert werden, nicht als Fluoreszenzphotonen emittiert, sondern die Energie wird auf die Quenchgruppe übertragen. Bei dieser Ausführungsform ist es daher zweckmäßig, die Positionen der Markierungsgruppe auf der Hybridisierungssonde bzw. der Quenchgruppe auf der Abfangsonde so zu wählen, dass bei einem Hybrid aus Hybridisierungssonde und Quenchgruppe beide Gruppen in räumlicher Nachbarschaft vorliegen. So kann beispielsweise die Markierungsgruppe am 5'-Ende der Hybridisierungssonde und die Quenchgruppe am 3'-Ende der Abfangsonde oder die Markierungsgruppe am 3'-Ende der Hybridisierungssonde und die Quenchgruppe am 5'-Ende der Abfangsonde angeordnet sein. Beispiele für Quenchgruppen, die das von einer Fluoreszenzmarkierungsgruppe erzeugte Signal zumindest teilweise löschen können, werden in US-Patent 5,607,834 offenbart.

In einer weiteren bevorzugten Ausführungsform können als Abfanggruppen auch Festphasenbindegruppen verwendet werden, die eine selektive Abtrennung der nicht an den Analyten gebundenen Markierungsgruppe durch Immobilisierung an einer geeigneten Festphase bewirken. Beispiele für Festphasenbindegruppen und damit bindefähigen Festphasen sind Bindepartner, die miteinander hochaffine Wechselwirkungen eingehen können, wie etwa Biotin (und Biotinanaloga) und Streptavidin oder Avidin bzw. ein Antigen oder ein Hapten und ein Antikörper bzw. ein Zucker und ein Lectin. In einer besonders bevorzugten Ausführungsform verwendet man eine biotinylierte Abfangsonde und eine mit Streptavidin oder Avidin beschichtete Festphase. Die Festphase kann beliebiger Natur sein, beispielsweise die Wand eines Reaktionsgefäßes, eine partikuläre Festphase, wie etwa Mikrobeads, oder eine von der Probe durchströmte Membran.

Ein wesentliches Merkmal des erfindungsgemäßen Verfahrens ist, dass ein Hybrid zwischen Abfangsonde und Hybridisierungssonde einen geringeren Schmelzpunkt als ein Hybrid zwischen Analyt und Hybridisierungssonde aufweist. Dieser Schmelzpunktunterschied beträgt unter Testbedingungen günstigerweise mindestens 1 °C, vorzugsweise mindestens 2 °C und besonders bevorzugt mindestens 5 °C. Die unterschiedliche Stabilität der Hybride kann beispielsweise dadurch erreicht werden, dass die Abfangsonde einen kürzeren; mit der Hybridisierungssonde komplementären Bereich als der Analyt oder/und mindestens einen Mismatch (d.h. eine nicht mit der Hybridisierungssonde komplementäre Base) in dem zum Analyten komplementären Bereich enthält.

Gemäß Schritt (d) des erfindungsgemäßen Verfahrens erfolgt das Inkontaktbringen von Probe und Hybridisierungssonde (in Anwesenheit oder Abwesenheit der Abfangsonde) unter ersten Hybridisierungsbedingungen, bei denen ein stabiles Hybrid zwischen Hybridisierungssonde und Analyt entstehen kann, aber bei denen kein stabiles Hybrid zwischen Hybridisierungssonde und Abfangsonde entstehen kann. Durch diesen ersten Hybridisierungsschritt werden diejenigen Hybridisierungssondenmoteküle, die mit den in der Probe vorhanden Analytmolekülen hybridisieren können, abreagiert, während die überzähligen Hybridisierungssondenmoleküle - in Anwesenheit oder in Abwesenheit der Abfangsonde - weiterhin in ungebundener Form vorliegen. Während des zweiten Hybridisierungsschritts (e) in Anwesenheit von Probe, Hybridisierungssonde und Abfangsonde werden zweite Hybridisierungsbedingungen eingestellt, z.B. durch Temperaturverringerung, bei denen auch ein stabiles Hybrid zwischen nicht an den Analyten gebundenen Hybridisierungssondenmolekülen und Abfangsondenmolekülen entstehen kann.

Es ist zweckmäßig, die Abfangsonden in ausreichender Menge zu verwenden, um nicht an den Analyten gebundene Hybridisierungssonden im Wesentlichen quantitativ zu binden, z.B. in einem molaren Überschuss von vorzugsweise mindestens ≥1,5:1, besonders bevorzugt ≥2:1 gegenüber den Hybridisierungssondenmolekülen.

Durch das Abfangen der Hybridisierungssonden wird das Hintergrundsignal der nicht an den Analyten gebundenen Markierungsgruppen beispielsweise durch Quenchen oder/und durch Abtrennen für eine Festphasenbindung selektiv verringert. Beim Nachweis der Bindung der Hybridisierungssonde an den Analyten gemäß (f) des erfindungsgemäßen Verfahrens kann somit eine signifikante Verringerung des unspezifischen Hintergrunds erzielt werden. Ein weiterer Vorteil ist die Vermeidung der unspezifischen Bindung von nicht an den Analyten gebundenen Hybridisierungssonden an weitere in der Probe vorhandene Nukleinsäuremoleküle.

In einer besonders bevorzugten Ausführungsform des Verfahrens verwendet man mindestens zwei mit dem Analyten bindefähige Hybridisierungssonden, die jeweils unterschiedliche Markierungsgruppen tragen, und jeweils eine Abfangsonde pro Hybridisierungssonde. In diesem Fall kann der Nachweis des Analyten durch gleichzeitige Bindung der mindestens zwei Hybridisierungssonden erfolgen.

Unterschiedliche Hybridisierungssonden beinhalten Markierungsgruppen, vorzugsweise Fluoreszenzmarkierungsgruppen, die sich mindestens in einem Messparameter, insbesondere ausgewählt aus Emissionswellenlänge und Dauer der Fluoreszenz, unterscheiden.

Die Bestimmung kann den Nachweis eines Energietransfers zwischen den Markierungsgruppen der ersten und zweiten Sonde umfassen. Beispielsweise enthält eine Sonde eine Donormarkierungsgruppe und die andere Sonde eine Akzeptormarkierungsgruppe, wobei ein Energietransfer zwischen Donor- und Akzeptormarkierungsgruppe stattfinden kann. Geeignete Kombinationen von Donor- und Akzeptormarkierungsgruppen für einen derartigen Energietransfer (FRET) sind dem Fachmann bekannt (siehe z.B. US-Patent 4,996,143).

Das erfindungsgemäße Verfahren kann jedoch auch eine sogenannte Kreuzkorrelationsbestimmung umfassen, bei der mindestens zwei unterschiedliche Markierungen, insbesondere Fluoreszenzmarkierungen, eingesetzt werden, deren korreliertes Signal bestimmt wird. Die grundsätzliche Durchführung einer solchen Kreuzkorrelationsbestimmung ist beispielsweise bei Schwille et al. (Biophys. J. 72 (1997), 1878-1886) und Rigler et al. (J. Biotechnol. 63 (1998), 97-109) beschrieben.

Eine Möglichkeit zur Verringerung von Interferenz bei der Kreuzkorrelation besteht darin, eine zeitaufgelöste Bestimmung durch Time-Gating durchzuführen. Hierbei werden die mindestens zwei an den Analyten gebundenen spektral unterschiedlichen Fluoreszenzmarkierungsgruppen repetitiv mit hoher Pulsfrequenz, die in der Größenordnung der Totzeit des Detektors liegt, z.B. im Bereich von 10 bis 0.1 MHz (entsprechend Taktintervallen von 0,1 bis 10 µs), angeregt. Dabei können durch entsprechende elektronische Hintergrundanalyse unter Berechnung einer Korrelations- oder/und Koinzidenzkurve "korrekt" auftretende Korrelationssignale von solchen Signalen unterschieden werden, die durch eine unerwünschte Interferenz (Crosstalk) beider Markierungsgruppen entstehen.

Das erfindungsgemäße Verfahren wird vorzugsweise mit geringen Konzentrationen des in der Probe vorhandenen Analyten bzw. der Hybridisierungssonden sowie mit einem sehr geringem Probenvolumen durchgeführt. Das Probenvolumen liegt vorzugsweise im Bereich von ≤ 10⁻⁶ I und besonders bevorzugt ≤ 10⁻⁸ I. Zur Bestimmung solcher Proben kann als Träger eine Mikrowellstruktur mit mehreren Vertiefungen zur Aufnahme von Probeflüssigkeit verwendet werden, wobei die einzelnen Wells beispielsweise einen Durchmesser zwischen 10 und 1 000 µm aufweisen. Geeignete Mikrostrukturen sind z.B. in DE 100 23 421.6 und DE 100 65 632.3 beschrieben. Weiterhin kann der Träger Temperatur-Steuerungselemente, z.B. Peltier-Elemente, beinhalten, die eine Temperaturregelung des Trägers oder/und einzelner Probebehältnisse darin ermöglichen.

Der für das Verfahren verwendete Träger ist zweckmäßigerweise so ausgestattet, dass er eine optische Detektion der Probe ermöglicht. Vorzugsweise wird daher ein zumindest im Bereich der Probenbehältnisse optisch transparenter Träger verwendet. Der Träger kann dabei entweder vollständig optisch transparent sein oder eine optisch transparente Basis und eine optisch undurchlässige Deckschicht mit Aussparungen in den Probenbehältnissen enthalten. Geeignete Materialien für Träger sind beispielsweise Verbundstoffträger aus Metall (z.B. Silizium für die Deckschicht) und Glas (für die Basis). Derartige Träger können beispielsweise durch Aufbringen einer Metallschicht mit vorgegebenen Aussparungen für die Probenbehältnisse auf das Glas erzeugt werden. Alternativ können Plastikträger, z.B. aus Polystyrol oder Polymeren auf Acrylat-oder Methacrylatbasis, eingesetzt werden. Weiterhin ist bevorzugt, dass der Träger eine Abdeckung für die Probenbehältnisse aufweist, um während der Messung ein geschlossenes und von der Umgebung im Wesentlichen isoliertes System bereitzustellen.

Weiterhin können im Träger elektrische Felder, insbesondere im Bereich der Probenbehältnisse, erzeugt werden, um eine Konzentrierung der zu bestimmenden Analyten im Messvolumen zu erreichen. Beispiele für Elektroden, die zur Erzeugung solcher elektrischer Felder geeignet sind, sind z.B. in DE 101 03 304.4 beschrieben.

Besonders bevorzugt erfolgt der Nachweis des Analyten durch Fluoreszenz-Korrelationsspektroskopie (FCS). Dabei erfolgt vorzugsweise die Messung von einem oder wenigen Analytmolekülen in einem Messvolumen, welches Teil des Probenvolumens ist, wobei die Konzentration der zu bestimmenden Analytmoleküle vorzugsweise ≤ 10⁻⁶ mol/l beträgt und das Messvolumen vorzugsweise ≤ 10⁻¹⁴ I ist. Es werden dabei von den Markierungsgruppen der Hybridisierungssonden stammende Messignale durch Lumineszenzmessung ermittelt. Auf Einzelheiten zu apparativen Details des Verfahrens und zur Durchführung des Verfahrens geeigneter Vorrichtungen wird auf EP-B-0 679 251 verwiesen.

Alternativ kann die Detektion auch durch zeitaufgelöste Abklingmessung, ein sogenanntes Time/Gating erfolgen, wobei das Signal der Markierungsgruppen, z.B. die Fluoreszenz, innerhalb des Messvolumens angeregt wird und anschließend vorzugsweise in einem zeitlichen Abstand von ≥100 ps das Öffnen des Detektionsintervalls am Detektor erfolgt. Diese Messmethode ist beispielsweise von Rigler et al. in "Ultrafast Phenomena" D.H. Auston, Hrsg., Springer 1984, beschrieben.

In einer besonders bevorzugten Ausführungsform des Verfahrens wird eine konfokale Einzelmolekülanalyse so durchgeführt, dass der Abstand zwischen dem Messvolumen in der Probenflüssigkeit und der Fokussieroptik der zur Anregung von Fluoreszenz-Markierungsgruppen in der Probenflüssigkeit verwendeten Lichtquelle ≥1 mm und die Probenflüssigkeit eine Lichtquelle, insbesondere von der Fokussier-Optik thermisch isoliert ist. Ein derartiges Verfahren und eine hierfür geeignete Vorrichtung ist beispielsweise in DE 101 11 420.6 beschrieben. Durch thermische Isolierung der Probenflüssigkeit von apparativen Komponenten kann die Temperatur der Probe unabhängig eingestellt und während des Verfahrens variiert werden. Somit können temperaturveränderliche Prozesse, z.B. die Bestimmung von Nukleinsäuren-Hybridisierungsschmelzkurven oder die Bestimmung von NukleinsäureAmplifikationsreaktionen, durchgeführt werden.

Der Träger ist vorzugsweise eine Mikrostruktur mit mehreren, vorzugsweise mindestens 10² Behältnissen zur Aufnahme einer Probenflüssigkeit, wobei die Probeflüssigkeit in den separaten Behältnissen aus einer oder mehreren Quellen stammen kann. Das Einbringen der Probeflüssigkeit in die Behältnisse des Trägers kann z.B. mittels einer piezoelektrischen Flüssigkeitsabgabe-Vorrichtung erfolgen.

Die Behältnisse des Trägers sind so ausgestaltet, dass sie eine Bindung des Nachweisreagenz mit dem Analyten in Lösung ermöglichen. Vorzugsweise handelt es sich bei den Behältnissen um Vertiefungen in der Trägeroberfläche, wobei diese Vertiefungen grundsätzlich eine beliebige Form aufweisen können, z.B. kreisförmig, quadratisch, rautenförmig etc. Der Träger kann auch 10³ oder mehr separate Behältnisse umfassen.

Die optische Anregungseinrichtung umfasst eine stark fokussierte Lichtquelle, vorzugsweise einen Laserstrahl, der mittels entsprechender optischer Einrichtungen auf das Messvolumen in der Probenflüssigkeit fokussiert wird. Die Lichtquelle kann auch zwei oder mehrere Laserstrahlen enthalten, die dann jeweils vor Eintritt in die Probenflüssigkeit durch unterschiedliche Optiken auf das Messvolumen fokussiert wird. Die Detektionseinrichtung kann beispielsweise einen fasergekoppelten Avalanche-Fotodiodendetektor oder einen elektronischen Detektor enthalten. Es können jedoch auch Anregungs- oder/und Detektions-Matrices, bestehend aus einer Punktmatrix von Laserpunkten, erzeugt durch eine Diffraktionsoptik oder einen Quanten-Well-Laser sowie einer Detektormatrix, erzeugt durch eine Avalanche-Fotodiodenmatrix oder elektronische Detektormatrix, z.B. eine CCD-Kamera, verwendet werden.

In einer bevorzugten Ausführungsform umfasst die Lichtquelle einen oder mehrere Laserstrahlen, die mittels Leiten durch ein oder mehrere diffraktionsoptische Elemente in multiple Foci aufgespalten werden, wie in DE 101 26 083.0 beschrieben.

Der Träger kann in vorgefertiger Form bereitgestellt werden, wobei in mehrere separate Behältnisse des Trägers lumineszenzmarkierte Nachweisreagenzien, vorzugsweise lumineszenzmarkierte Hybridisierungssonden bzw. -primer, eingefüllt werden. Anschließend wird der die Nachweisreagenzien enthaltende Träger günstigerweise getrocknet.

In einer bevorzugten Ausführungsform der Erfindung wird ein vorgefertigter Träger bereitgestellt, der eine Vielzahl von separaten, z.B. 100 Behältnissen enthält, in die jeweils unterschiedliche Nachweisreagenzien, z.B. Reagenzien zum Nachweis einer Nukleinsäurehybridisierung, wie Primer oder/und Sonden, eingefüllt vorliegen. Dieser Träger kann dann mit einer aus einem zu untersuchenden Organismus, z.B. einem menschlichen Patienten, stammenden Probe gefüllt werden, so dass in den jeweiligen Behältnissen unterschiedliche Analyten aus einer einzigen Probe bestimmt werden. Derartige Träger können beispielsweise zur Erstellung eines Genexpressionsprofils, z.B. für die Diagnostik von Krankheiten, oder zur Bestimmung von Nukleinsäurepolymorphismen, z.B. zum Nachweis einer bestimmten genetischen Prädisposition, verwendet werden.

Schließlich betrifft die Erfindung ein Reagenzienkit zum Nachweis von Nukleinsäure-Analyten umfassend
(a) mindestens eine mit einem Analyten bindefähige Hybridisierungssonde, die eine Markierungsgruppe trägt, und
(b) mindestens eine mit der Hybridisierungssonde bindefähigen Abfangsonde, wobei die Abfangsonde mindestens eine Abfanggruppe trägt, die bei Bindung an die Hybridisierungssonde eine Verringerung des von der Markierungsgruppe stammenden Signals bewirkt und wobei ein Hybrid zwischen Abfangsonde und Hybridisierungssonde einen geringeren Schmelzpunkt als ein Hybrid zwischen Analyt und Hybridisierungssonde aufweist.

Dieser Reagenzienkit wird vorzugsweise in einem Nachweisverfahren, wie zuvor beschrieben, eingesetzt.

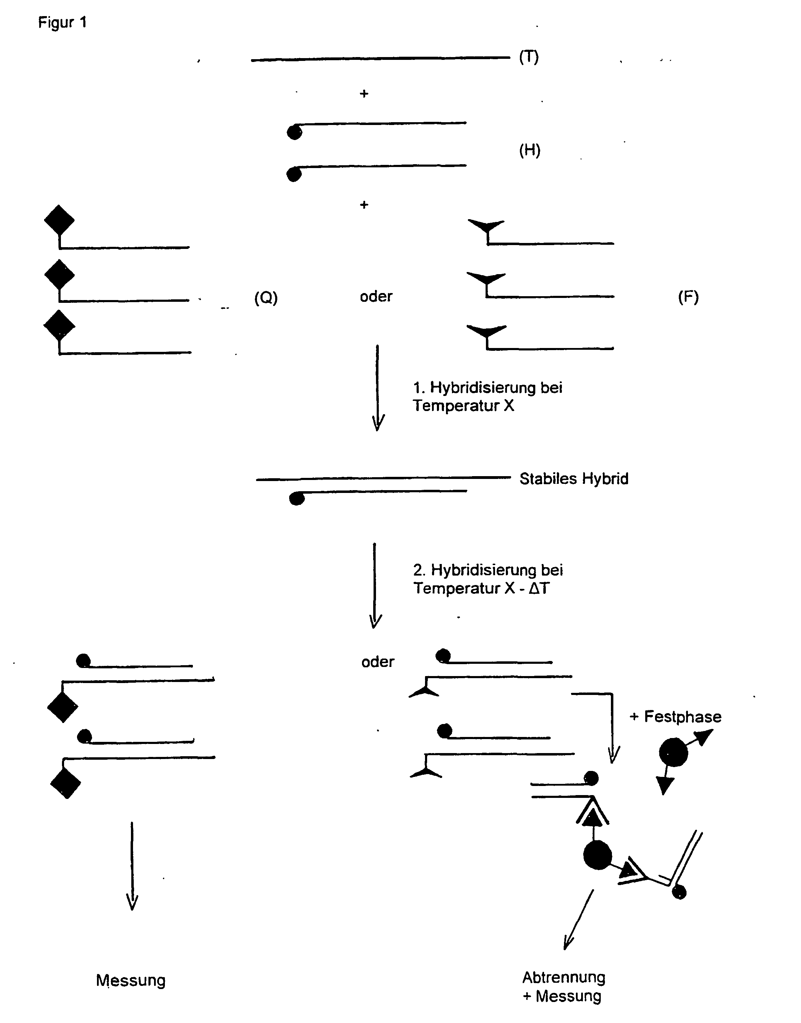

Figur 1 zeigt eine bevorzugt Ausführungsform der vorliegenden Erfindung. Eine Probe mit einer darin enthaltenen Zielnukleinsäure (T) wird mit einer Hybridisierungssonde (H) und einer Abfangsonde enthaltend eine Quenchgruppe (Q) oder eine Festphasenbindegruppe (F) in Kontakt gebracht. Zunächst erfolgt eine erste Hybridisierung bei einer Temperatur X, bei der ein stabiles Hybrid zwischen Zielnukleinsäure (T) und Hybridisierungssonde (H) entsteht, bei der aber keine stabilen Hybridisierungsprodukte zwischen den Abfangsonden (Q oder F) und der Hybridisierungssonde (H) entstehen können.

Nach einer ausreichenden Inkubationsdauer erfolgt eine zweite Hybridisierung bei einer Temperatur X - ^{Δ}T, wobei stabile Hybridisierungsprodukte zwischen Hybridisierungssonde (H) und den Abfangsonden (Q bzw. F) entstehen können. Im Falle, dass Abfangsonden (F) mit Festphasenbindegruppen verwendet werden, muss eine Abtrennung der zweiten Hybridisierungsprodukte von den ersten Hybridisierungsprodukten durch Festphasenbindung, z.B. durch Zugabe von geeigneten beschichteten Beads und nachfolgende Zentrifugation, erfolgen.

Schließlich kann der Nachweis des ersten Hybridisierungsprodukts aus Zielnukleinsäure (T) und Hybridisierungssonde (A) erfolgen, ohne dass überschüssige freie Hybridisierungssondenmolekülediesen Nachweis stören würden.

## Patentansprüche

1. Verfahren zur Bestimmung eines Nukleinsäure-Analyten in einer Probe, umfassend die Schritte:
(a) Bereitstellen einer Probe, die den Analyten enthalten kann,
(b) Bereitstellen von mindestens einer mit dem Analyten bindefähigen Hybridisierungssonde, die eine Markierungsgruppe trägt,
(c) Bereitstellen von mindestens einer mit der Hybridisierungssonde bindefähigen Abfangsonde, wobei die Abfangsonde mindestens eine Abfanggruppe trägt, die bei Bindung an die Hybridisierungssonde eine Verringerung des von der Markierungsgruppe stammenden Signals bewirkt und wobei ein Hybrid zwischen Abfangsonde und Hybridisierungssonde einen geringeren Schmelzpunkt als ein Hybrid zwischen Analyt und Hybridisierungssonde aufweist,
(d) Inkontaktbringen von Probe und Hybridisierungssonde unter ersten Hybridisierungsbedingungen, bei denen ein stabiles Hybrid zwischen Hybridisierungssonde und Analyt entstehen kann, aber bei denen kein stabiles Hybrid zwischen Hybridisierungssonde und Abfangsonde entstehen kann,
(e) Inkontaktbringen von Probe, Hybridisierungssonde und Abfangsonde unter zweiten Hybridisierungsbedingungen, bei denen ein stabiles Hybrid zwischen nicht an den Analyten gebundener Hybridisierungssonde und Abfangsonde entstehen kann und wobei durch Bindung der Hybridisierungssonde an die Abfangsonde eine Verringerung des von deren Markierungsgruppe stammenden Signals bewirkt wird, und
(f) Nachweisen der Bindung der Hybridisierungssonde an den Analyten.

2. Verfahren nach Anspruch 1,
**dadurch gekennzeichnet,**
**dass** man Hybridisierungssonden verwendet, die eine Fluoreszenzmarkierungsgruppe tragen.

3. Verfahren nach Anspruch 1 oder 2,
**dadurch gekennzeichnet,**
**dass** man als Abfanggruppe eine das Signal der Markierungsgruppe zumindest teilweise löschende Quenchgruppe verwendet.

4. Verfahren nach Anspruch 1 oder 2,
**dadurch gekennzeichnet,**
**dass** man als Abfanggruppe eine Festphasenbindegruppe verwendet und die an die Abfangsonde gebundene Hybridisierungssonde durch Immobilisierung an einer Festphase abtrennt.

5. Verfahren nach Anspruch 4,
**dadurch gekennzeichnet,**
**dass** man eine biotinylierte Abfangsonde und eine Streptavidin- oder Avidin-beschichtete Festphase verwendet.

6. Verfahren nach einem der vorhergehenden Ansprüche,
**dadurch gekennzeichnet,**
**dass** die Abfangsonde einen kürzeren mit der Hybridisierungssonde komplementären Bereich als der Analyt oder/und mindestens einen Mismatch in den zum Analyten komplementären Bereich trägt.

7. Verfahren nach einem der vorhergehenden Ansprüche,
**dadurch gekennzeichnet,**
**dass** der Schmelzpunktunterschied des Hybrids zwischen Abfangsonde und Hybridisierungssonde und des Hybrids zwischen Analyt und Hybridisierungssonde mindestens 1 °C, vorzugsweise mindestens 2 °C und besonders bevorzugt mindestens 5 °C unter Testbedingungen beträgt.

8. Verfahren nach einem der vorhergehenden Ansprüche,
**dadurch gekennzeichnet,**
**dass** man mindestens zwei mit dem Analyten bindefähige Hybridisierungsonden, die jeweils unterschiedliche Markierungsgruppen tragen, und jeweils eine Abfangsonde pro Hybridisierungssonde verwendet.

9. Verfahren nach Anspruch 8,
**dadurch gekennzeichnet,**
**dass** man den Analyten durch gleichzeitige Bindung der mindestens zwei Hybridisierungssonden nachweist.

10. Verfahren nach einem der vorhergehenden Ansprüche,
**dadurch gekennzeichnet,**
**dass** Schritt (f) einen Nachweis durch konfokale Detektion umfasst.

11. Verfahren nach Anspruch 10,
**dadurch gekennzeichnet,**
**dass** der Nachweis eine Einzelmoleküldetektion umfasst.

12. Verfahren nach einem der Ansprüche 10 oder 11,
**dadurch gekennzeichnet,**
**dass** Schritt (f) einen Nachweis durch Fluoreszenz-Korrelationsspektroskopie oder/und Time-Gating umfasst.

13. Verfahren nach einem der Ansprüche 10 bis 12,
**dadurch gekennzeichnet,**
**dass** man eine einzige Lichtquelle zur Anregung der Markierungsgruppe(n) verwendet.

14. Verfahren nach einem der Ansprüche 10 bis 12,
**dadurch gekennzeichnet,**
**dass** man mehrere Lichtquellen zur Anregung der Markierungsgruppe(n) verwendet.

15. Verfahren nach einem der Ansprüche 2 bis 14,
**dadurch gekennzeichnet,**
**dass** die zur Anregung der Markierungsgruppen verwendete(n) Lichtquelle(n) in Form von repetitiven Pulsen in die Probe eingestrahlt wird (werden).

16. Verfahren nach Anspruch 15,
**dadurch gekennzeichnet,**
**dass** die Frequenz der Pulse im Bereich von 10 bis 0,1 MHz liegt.

17. Reagenzienkit zum Nachweis von Nukleinsäureanalyten, umfassend:
(a) mindestens eine mit einem Analyten bindefähige Hybridisierungssonde, die eine Markierungsgruppe trägt, und
(b) mindestens eine mit der Hybridisierungssonde bindefähigen Abfangsonde, wobei die Abfangsonde mindestens eine Abfanggruppe trägt, die bei Bindung an die Hybridisierungssonde eine Verringerung des von der Markierungsgruppe stammenden Signals bewirkt und wobei ein Hybrid zwischen Abfangsonde und Hybridisierungssonde einen geringeren Schmelzpunkt als ein Hybrid zwischen Analyt und Hybridisierungssonde aufweist.

## Claims

1. A method for determining a nucleic acid analyte in a sample, comprising the steps:
(a) preparing a sample which may contain the analyte,
(b) preparing at least one hybridisation probe which is able to bind to the analyte and carries a labelling group,
(c) preparing at least one capture probe able to bind to the hybridisation probe, wherein the capture probe carries at least one capture group which, on binding to the hybridisation probe, brings about a reduction in the signal derived from the labelling group, and wherein a hybrid between capture probe and hybridisation probe has a lower melting point than a hybrid between analyte and hybridisation probe,
(d) contacting sample and hybridisation probe under first hybridisation conditions, under which a stable hybrid is possible between hybridisation probe and analyte but under which no stable hybrid can be formed between hybridisation probe and capture probe,
(e) contacting sample, hybridisation probe and capture probe under second hybridisation conditions, under which a stable hybrid is possible between hybridisation probe not bound to the analyte, and capture probe, and wherein through binding of the hybridisation probe to the capture probe a reduction is brought about in the signal derived from the labelling group thereof, and
(f) detection of the binding of the hybridisation probe to the analyte.

2. A method as in claim 1, **characterised in that** hybridisation probes which carry a fluorescence labelling group are used.

3. A method as in claim 1 or 2, **characterised in that** a quencher group which at least partly quenches the signal of the labelling group is used as capture group.

4. A method as in claim 1 or 2, **characterised in that** a solid phase binding group is used as capture group, and the hybridisation probe bound to the capture probe is removed by immobilisation on a solid phase.

5. A method as in claim 4, **characterised in that** a biotinylated capture probe and a streptavidin or avidin-coated solid phase are used.

6. A method as in any of the preceding claims, **characterised in that** the capture probe has a shorter region complementary to the hybridisation probe than the analyte or/and has at least one mismatch in the region complementary to the analyte.

7. A method as in any of the preceding claims, **characterised in that** the melting point difference of the hybrid between capture probe and hybridisation probe and of the hybrid between analyte and hybridisation probe is at least 1°C, preferably at least 2°C and particularly preferably at least 5°C under assay conditions.

8. A method as in any of the preceding claims, **characterised in that** at least two hybridisation probes which are able to bind to the analyte and each of which carry different labelling groups, and in each case one capture probe per hybridisation probe, are used.

9. A method as in claim 8, **characterised in that** the analyte is detected by simultaneous binding of the at least two hybridisation probes.

10. A method as in any of the preceding claims, **characterised in that** step (f) includes a detection by confocal detection.

11. A method as in claim 10, **characterised in that** the detection includes a single molecule detection.

12. A method as in either of claims 10 or 11, **characterised in that** step (f) includes a detection by fluorescence correlation spectroscopy or/and time gating.

13. A method as in any of claims 10 to 12, **characterised in that** a single light source is used to excite the labelling group(s).

14. A method as in any of claims 10 to 12, **characterised in that** a plurality of light sources is used to excite the labelling group(s).

15. A method as in any of claims 2 to 14, **characterised in that** the light source(s) used to excite the labelling groups is (are) beamed into the sample in the form of repetitive pulses.

16. A method as in claim 15, **characterised in that** the frequency of the pulses is in the range from 10 to 0.1 MHz.

17. A reagent kit for detecting nucleic acid analytes, comprising:
(a) at least one hybridisation probe which is able to bind to an analyte and carries a labelling group, and
(b) at least one capture probe able to bind to the hybridisation probe, wherein the capture probe carries at least one capture group which, on binding to the hybridisation probe, brings about a reduction in the signal derived from the labelling group, and wherein a hybrid between capture probe and hybridisation probe has a lower melting point than a hybrid between analyte and hybridisation probe.

## Revendications

1. Procédé pour déterminer un analyte d'acide nucléique dans un échantillon, comprenant les étapes :
(a) préparation d'un échantillon qui peut contenir l'analyte,
(b) préparation d'au moins une sonde d'hybridation capable de se lier avec l'analyte, qui porte un groupe de marquage,
(c) préparation d'au moins une sonde de fixation capable de se lier avec la sonde d'hybridation, où la sonde de fixation porte au moins une groupe de fixation qui, lors de la liaison à la sonde d'hybridation, provoque une diminution du signal provenant du groupe de marquage et où un hybride entre la sonde de fixation et la sonde d'hybridation présente un point de fusion plus bas qu'un hybride entre l'analyte et la sonde d'hybridation,
(d) mise en contact de l'échantillon et de la sonde d'hybridation dans des premières conditions d'hybridation dans lesquelles un hybride stable peut se former entre la sonde d'hybridation et l'analyte, mais dans lesquelles aucun hybride stable ne peut se former entre la sonde d'hybridation et la sonde de fixation,
(e) mise en contact de l'échantillon, de la sonde d'hybridation et de la sonde de fixation dans des secondes conditions d'hybridation dans lesquelles un hybride stable peut se former entre la sonde d'hybridation non liée à l'analyte et la sonde de fixation et où, par liaison de la sonde d'hybridation à la sonde de fixation, il se produit une diminution du signal provenant de son groupe de marquage, et
(f) mise en évidence de la liaison de la sonde d'hybridation à l'analyte.

2. Procédé selon la revendication 1 **caractérisé en ce que** l'on utilise des sondes d'hybridation qui portent un groupe de marquage par fluorescence.

3. Procédé selon la revendication 1 ou 2 **caractérisé en ce que** l'on utilise comme groupe de fixation un groupe d'extinction éteignant au moins partiellement le signal du groupe de marquage.

4. Procédé selon la revendication 1 ou 2 **caractérisé en ce que** l'on utilise comme groupe de fixation un groupe de liaison à une phase solide et on sépare par immobilisation à une phase solide la sonde d'hybridation liée à la sonde de fixation.

5. Procédé selon la revendication 4 **caractérisé en ce que** l'on utilise une sonde de fixation biotinylée et une phase solide recouverte de streptavidine ou d'avidine.

6. Procédé selon l'une des revendications précédentes **caractérisé en ce que** la sonde de fixation porte un domaine complémentaire de la sonde d'hybridation plus court que l'analyte et/ou au moins un mésappariement dans le domaine complémentaire de l'analyte.

7. Procédé selon l'une des revendications précédentes **caractérisé en ce que** la différence de point de fusion de l'hybride entre la sonde de fixation et la sonde d'hybridation et de l'hybride entre l'analyte et la sonde d'hybridation est d'au moins 1 °C, de préférence d'au moins 2°C et de manière particulièrement préférée d'au moins 5°C dans les conditions du test.

8. Procédé selon l'une des revendications précédentes **caractérisé en ce que** l'on utilise au moins deux sondes d'hybridation capables de se lier avec l'analyte, qui portent dans chaque cas des groupes de marquage différents, et dans chaque cas une sonde de fixation par sonde d'hybridation.

9. Procédé selon la revendication 8 **caractérisé en ce que** l'on met l'analyte en évidence par liaison simultanée des deux sondes d'hybridation au moins.

10. Procédé selon l'une des revendications précédentes **caractérisé en ce que** l'étape (f) comprend une mise en évidence par détection confocale.

11. Procédé selon la revendication 10 **caractérisé en ce que** la mise en évidence comprend une détection de molécule unique.

12. Procédé selon l'une des revendications 10 ou 11 **caractérisé en ce que** l'étape (f) comprend une mise en évidence par spectroscopie à corrélation de fluorescence et/ou time-gating.

13. Procédé selon l'une des revendications 10 à 12 **caractérisé en ce que** l'on utilise une seule source lumineuse pour l'excitation du ou des groupes de marquage.

14. Procédé selon l'une des revendications 10 à 12 **caractérisé en ce que** l'on utilise plusieurs sources lumineuses pour l'excitation du ou des groupes de marquage.

15. Procédé selon l'une des revendications 2 à 14 **caractérisé en ce que** la ou les sources lumineuses utilisées pour l'excitation des groupes de marquage sont projetées dans l'échantillon sous forme d'impulsions répétitives.

16. Procédé selon la revendication 15 **caractérisé en ce que** la fréquence des impulsions est située dans le domaine de 10 à 0,1 MHz.

17. Kit de réactifs pour la mise en évidence d'analytes d'acide nucléique, comprenant :
(a) au moins une sonde d'hybridation capable de se lier avec l'analyte, qui porte un groupe de marquage, et
(b) au moins une sonde de fixation capable de se lier avec la sonde d'hybridation, où la sonde de fixation porte au moins un groupe de fixation qui, lors de la liaison à la sonde d'hybridation, provoque une diminution du signal provenant du groupe de marquage et où un hybride entre la sonde de fixation et la sonde d'hybridation présente un point de fusion plus bas qu'un hybride entre l'analyte et la sonde d'hybridation.
